# EUROPEAN PATENT APPLICATION

(11) **EP 1 741 694 A1**
(43) Date of publication of application: **10.01.2007**
(21) Application number: 05736827.6
(22) Date of filing: 26.04.2005
(51) Int. Cl.: C07C 29/50, C07C 35/08, C07B 61/00

(54) **METHOD FOR PRODUCING CYCLOALKANOL AND/OR CYCLOALKANONE**

(30) Priority: 30.04.2004 JP 2004135495
(71) Applicant: Sumitomo Chemical Company, Limited, Tokyo 104-8260 (JP)
(72) Inventor: BATTIONI, Pierrette, F-91300 (FR); MANSUY, Daniel, F-75015 (FR); ISHIDA, Hajime, 7920025 (JP)
(74) Representative: Hubert, Philippe
(86) International application number: PCT/JP2005/007871
(87) International publication number: WO 2005/105716

(57) **Abstract**

Cycloalkanol and/or cycloalkanone is produced by oxidizing cycloalkane with molecular oxygen in the presence of a compound represented by the formula (1): 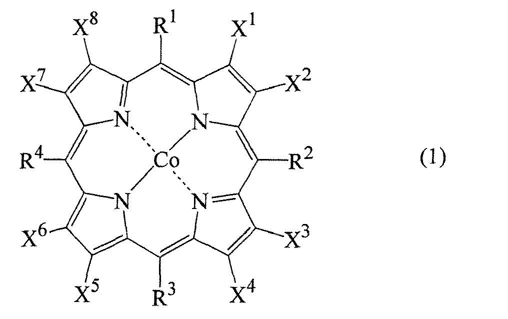 wherein X¹ to X⁸ independently represent a hydrogen atom, a halogen atom, a nitro group, a cyano group, a hydrocarbon group, a halogenated hydrocarbon group or a sulfonyl group, provided that at least one of X¹ to X⁸ is a halogen atom, and R¹ to R⁴ independently represent a hydrogen atom, a nitro group, a cyano group, a hydrocarbon group or a halogenated hydrocarbon group.

## Description

### Technical Field

The invention relates to a process for producing cycloalkanol and/or cycloalkanone by oxidizing cycloalkane with molecular oxygen. The resulting cycloalkanol can be easily converted into cycloalkanone by a known method, and cycloalkanone is used as the starting material for producing oxime or lactam.

### Background Art

It is known that a porphyrin complex is used as a catalyst in a method of oxidizing hydrocarbon such as alkane, cycloalkane or alkene with molecular oxygen. As such a catalyst, for instance, JP-A 5-138037 discloses a complex of iron, chromium, manganese, ruthenium or copper with perhalogenated porphyrin as a ligand, and JP-A 5-200302 discloses a complex of iron, chromium, manganese, ruthenium, cobalt or copper with porphyrin cyanide as a ligand. Further, JP-A 5-262684 discloses a complex of iron, chromium, manganese, ruthenium, cobalt or copper with nitrated porphyrin as a ligand, and JP-A 8-104658 discloses a complex of iron with halogenated porphyrin as a ligand.

### Disclosure of Invention

The above-described conventional methods, however, did not always provide sufficient activity of a catalyst and sufficient selectivity to the aimed product, in particular, in producing cycloalkanol and/or cycloalkanone by oxidizing cycloalkane with molecular oxygen. Therefore, an object of the present invention is to provide a process for producing cycloalkanol and/or cycloalkanone in high selectivity by oxidizing cycloalkane with molecular oxygen at a high conversion rate.

That is, the present invention provides a process for producing cycloalkanol and/or cycloalkanone, which comprises oxidizing cycloalkane with molecular oxygen in the presence of a compound represented by the formula (1): 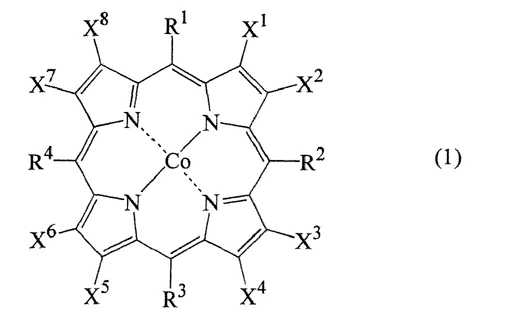 wherein X¹ to X⁸ independently represent a hydrogen atom, a halogen atom, a nitro group, a cyano group, a hydrocarbon group, a halogenated hydrocarbon group or a sulfonyl group, provided that at least one of X¹ to X⁸ is a halogen atom, and R¹ to R⁴ independently represent a hydrogen atom, a nitro group, a cyano group, a hydrocarbon group or a halogenated hydrocarbon group.

### Effect of Invention

According to the present invention, cycloalkane can be oxidized at a high conversion rate to produce cycloalkanol and/or cycloalkanone in high selectivity.

### Best Mode for Carrying Out the Invention

The present invention will be described in detail hereinafter. In the present invention, cycloalkane is used as the starting material, which is oxidized with molecular oxygen in the presence of a catalyst to produce the corresponding cycloalkanol and/or cycloalkanone.

Examples of cycloalkane as the starting material include monocyclic cycloalkane having no substituent on the ring, such as cyclopropane, cyclobutane, cyclopentane, cyclohexane, cycloheptane, cyclooctane, cyclodecane, and cyclooctadecane; polycyclic cycloalkane such as norbornane, decalin, and adamantane; and cycloalkane having a substituent on the ring, such as methylcyclopentane and methylcyclohexane. The cycloalkane usually has about 3 to 20 carbon atoms. Two or more cycloalkane may be used, if necessary.

An oxygen-containing gas is usually used as a source of molecular oxygen. For instance, the oxygen-containing gas may be air, pure oxygen, or air or pure oxygen diluted with inert gas such as nitrogen, argon or helium. Alternatively, oxygen-enriched air which is obtained by adding pure oxygen to air can be used.

In the present invention, a compound represented by the above formula (1) (hereinafter, referred to as "compound (1)") is used as a catalyst for oxidizing cycloalkane with molecular oxygen. By using such a cobalt porphyrin complex as a catalyst, the oxidation of cycloalkane can be smoothly progressed to produce cycloalkanol and/or cycloalkanone in high selectivity.

When at least one of X¹ to X⁸ present on pyrrole rings in the formula (1) is a hydrocarbon group, the hydrocarbon group can be an aliphatic hydrocarbon group, an alicyclic hydrocarbon group or an aromatic hydrocarbon group.

The aliphatic hydrocarbon group, when used herein, is a residue obtained by removing a hydrogen atom from aliphatic hydrocarbon and usually has about 1 to 20 carbon atoms. Specific examples of the aliphatic hydrocarbon groups include alkyl groups such as a methyl group, an ethyl group, a n-propyl group, an iso-propyl group, a n-butyl group, an isobutyl group, a s-butyl group and a t-butyl group; alkenyl groups such as a vinyl group, an allyl group and a methallyl group; and alkynyl groups such an ethynyl group and a propargyl group.

The alicyclic hydrocarbon group is a residue obtained by removing a hydrogen atom from alicyclic hydrocarbon and usually has about 3 to 20 carbon atoms. The alicyclic hydrocarbon group may be a residue obtained by removing a hydrogen atom from the aliphatic ring of alicyclic hydrocarbon, or a residue obtained by removing a hydrogen atom from the aliphatic chain of aliphatic chain-containing alicyclic hydrocarbon. Specific examples of the alicyclic hydrocarbon group include cycloalkyl groups such as a cyclopropyl group, a cyclobutyl group, a cyclopentyl group and a cyclohexyl group; and cycloalkylalkyl groups such as a cyclopentylmethyl group and a cyclohexylmethyl group.

The aromatic hydrocarbon group is a residue obtained by removing a hydrogen atom from aromatic hydrocarbon and usually has about 6 to 20 carbon atoms. The aromatic hydrocarbon group may be a residue obtained by removing a hydrogen atom from the aromatic ring of aromatic hydrocarbon, a residue obtained by removing a hydrogen atom from the aliphatic chain of aliphatic chain-containing aromatic hydrocarbon, or a residue obtained by removing a hydrogen atom from the aliphatic ring of aliphatic ring-containing aromatic hydrocarbon. Specific examples of the aromatic hydrocarbon group include aryl groups such as a phenyl group, a tolyl group and a naphthyl group; and arylalkyl groups (aralkyl groups) such as a benzyl group and a phenethyl group.

When at least one of X¹ to X⁸ is a halogenated hydrocarbon group, the halogenated hydrocarbon group can be a group obtained by substituting at least one hydrogen atom of a hydrocarbon group with a fluorine atom, a chlorine atom, a bromine atom or an iodine atom. When at least one of X¹ to X⁸ is a sulfonyl group, the sulfonyl group can be a group obtained by bonding a hydrocarbon group to a unsubstituted sulfonyl group (-SO₂-). The hydrocarbon group for the halogenated hydrocarbon group and the sulfonyl group can be an aliphatic hydrocarbon group, an alicyclic hydrocarbon group or an aromatic hydrocarbon group, as described above for a hydrocarbon group represented by X¹ to X⁸, and examples thereof are also the same as the examples of a hydrocarbon group represented by X¹ to X⁸.

When at least one of R¹ to R⁴ present on carbon atoms connecting pyrrole rings in the formula (1) is a hydrocarbon group, the hydrocarbon group can be an aliphatic hydrocarbon group, an alicyclic hydrocarbon group or an aromatic hydrocarbon group, as described above for a hydrocarbon group represented by X¹ to X⁸, and examples thereof are also the same as the examples of a hydrocarbon group represented by X¹ to X⁸.

When at least one of R¹ to R⁴ is a halogenated hydrocarbon group, the halogenated hydrocarbon group can be a group obtained by substituting at least one hydrogen atom of a hydrocarbon group with a fluorine atom, a chlorine atom, a bromine atom or an iodine atom, as described above for a halogenated hydrocarbon group represented by X¹ to X⁸. The hydrocarbon group for the halogenated hydrocarbon group can be an aliphatic hydrocarbon group, an alicyclic hydrocarbon group or an aromatic hydrocarbon group, as described above for a hydrocarbon group represented by X¹ to X⁸, and examples thereof are also the same as the examples of a hydrocarbon group represented by X¹ to X⁸.

As described above, at least one of X¹ to X⁸ is a halogen atom. The others are preferably independently a hydrogen atom, a hydrocarbon group or a halogenated hydrocarbon group. More preferably, all of X¹ to X⁸ are halogen atoms. R¹ to R⁴ are preferably independently a hydrogen atom, a hydrocarbon group or a halogenated hydrocarbon group.

Compound (1) can be prepared by a known method, for instance, a method described in Journal of Organic Chemistry, volume 32, p.476 (1967), or a method described in Inorganic Chemistry, volume 31, p.2044 (1992). Compound (1) may be used in the supported form on a carrier such as silica, alumina, titania, montmorillonite, zeolite or hydrotalcite.

The oxidation reaction of cycloalkane in the present invention can be conducted by contacting cycloalkane with molecular oxygen in the presence of compound (1). The amount of compound (1) to be used is usually 0.01 to 100 ppm by weight, preferably 0.1 to 50 ppm by weight, based on the amount of cycloalkane. The reaction temperature is usually 0 to 200°C, preferably 100 to 180°C. The reaction pressure is usually 0.01 to 10 MPa, preferably 0.1 to 2 MPa. A solvent may be used in the reaction, if necessary. Examples of the solvent include nitrile solvents such as acetonitrile and benzonitrile; and carboxylic acid solvents such as acetic acid and propionic acid.

Post-treatment of the oxidation reaction includes, but not particularly limited to, washing of the reaction mixture with water to separate compound (1), followed by distillation. When the reaction mixture contains cycloalkylhydroperoxide corresponding to the starting material cycloalkane, the cycloalkylhydroperoxide can be converted into the aimed products, cycloalkanol or cycloalkanone, by alkali treatment, reduction processing or the like.

### Example

Hereinafter, the present invention will be illustrated by referring to Examples which the present invention is not limited to. Cyclohexane, cyclohexanone, cyclohexanol and cyclohexylhydroperoxide in a reaction mixture were analyzed by gas chromatography. Based on the analysis results, the conversion rate of cyclohexane, and selectivity to cyclohexanone, cyclohexanol and cyclohexylhydroperoxide were calculated.

### Example 1

Cobalt(II) 5,10,15,20-tetrakis (pentafluorophenyl)-2,3,7,8,12,13,17,18-octabromoporphyrin [a compound (1) wherein X¹ to X⁸ are bromine atoms and R¹ to R⁴ are pentafluorophenyl groups] (0.0548 g, 3.3×10⁻⁵ mol) as a catalyst was dissolved in cyclohexane (2221.8 g, 26.4 mol) to prepare a supply solution (catalyst/ starting material solution). After 278 g of the supply solution was put in a 1 L autoclave, the autoclave was pressurized to 0.6 MPa with nitrogen and then heated to 125°C while passing nitrogen. Into the autoclave, the supply solution was further supplied at a rate of 4.6 g/minute, while air was passed so that the oxygen concentration in exhaust gas was 1-5% by volume. Continuous reaction was performed for 4 hours under the reaction temperature of 125°C and the residence time of 1 hour. Then, the resulting reaction mixture was analyzed. As a result, the conversion rate of cyclohexane was 2.3%, the cyclohexanone-selectivity was 33.5%, the cyclohexanol-selectivity was 50.3%, and the cyclohexylhydroperoxide-selectivity was 3.9%.

### Comparative Example 1

The same procedure as Example 1 was performed except that 0.0222 g (3.3x10⁻⁵ mol) of cobalt (II) 5,10,15,20-tetraphenylporphyrin [a compound (1) wherein X¹ to X⁸ are hydrogen atoms and R¹ to R⁴ are phenyl groups] was used as a catalyst. The conversion rate of cyclohexane was 0.8%, the cyclohexanone-selectivity was 26.1%, the cyclohexanol-selectivity was 39.0%, and the cyclohexylhydroperoxide-selectivity was 24.1%.

### Comparative Example 2

The same procedure as Example 1 was performed except that 0.0261 g (3.3×10⁻⁵ mol) of cobalt(II) 5,10,15,20-tetrakis(p-methoxyphenyl)porphyrin [a compound (1) wherein X¹ to X⁸ are hydrogen atoms and R¹ to R⁴ are p-methoxyphenyl groups] was used as a catalyst. The conversion rate of cyclohexane was 0.9%, the cyclohexanone-selectivity was 31.2%, the cyclohexanol-selectivity was 32.1%, and the cyclohexylhydroperoxide-selectivity was 27.8%.

### Comparative Example 3

The same procedure as Example 1 was performed except that 0.0633 g (3.7×10⁻⁵ mol) of iron(III) 5,10,15,20-tetrakis-2,3,7,8,12,13,17,18-(pentafluorophenyl)octabromoporphyrin chloride [a compound (1) wherein X¹ to X⁸ are bromine atoms, R¹ to R⁴ are pentafluorophenyl groups, and Fe-Cl was substituted for Co] as a catalyst was dissolved in 2499.6 g (29.7 mol) of cyclohexane to prepare a supply solution. The conversion rate of cyclohexane was 0.9%, the cyclohexanone-selectivity was 25.4%, the cyclohexanol-selectivity was 32.0%, and the cyclohexylhydroperoxide-selectivity was 20.8%.

### Examples 2-11

In each Example, the same procedure as Example 1 was performed except that 0.0070 g (4.2×10⁻⁶ mol) of cobalt(II) 5,10,15,20-tetrakis-2,3,7,8,12,13,17,18-(pentafluorophenyl)octabromoporphyrin as a catalyst was dissolved in 2499.6 g (29.7 mol) of cyclohexane to prepare a supply solution and the reaction was conducted under the reaction temperature and the residence time shown in Table 1. The results, that is, the conversion rate of cyclohexane, the cyclohexanone-selectivity, the cyclohexanol-selectivity and the cyclohexylhydroperoxide-selectivity are shown in Table 1.

**Table 1**

| Example No. | Reaction Temperature (°C) | Residence Time (min) | Conversion Rate of Cyclohexane (%) | Cycloheanone-Selectivity (%) | Cyclohexanol-Selectivity (%) | Cyclohexylhydroperoxide-Selectivity (%) |
|---|---|---|---|---|---|---|
| 2 | 125 | 60 | 0.7 | 17.5 | 36.3 | 29.7 |
| 3 | 130 | 60 | 0.9 | 16.6 | 40.8 | 29.8 |
| 4 | 135 | 60 | 2.1 | 23.2 | 44.1 | 20.4 |
| 5 | 135 | 90 | 3.7 | 31.4 | 43.9 | 10.9 |
| 6 | 135 | 120 | 4.4 | 36.6 | 33.2 | 7.3 |
| 7 | 140 | 60 | 3.3 | 27.6 | 40.0 | 18.9 |
| 8 | 140 | 75 | 5.3 | 31.6 | 40.9 | 10.4 |
| 9 | 140 | 90 | 6.5 | 31.7 | 36.4 | 13.7 |
| 10 | 145 | 60 | 5.5 | 29.4 | 38.3 | 15.7 |
| 11 | 145 | 75 | 7.5 | 31.7 | 35.7 | 10.7 |

## Claims

1. A process for producing cycloalkanol and/or cycloalkanone, which comprises oxidizing cycloalkane with molecular oxygen in the presence of a compound represented by the formula (1): 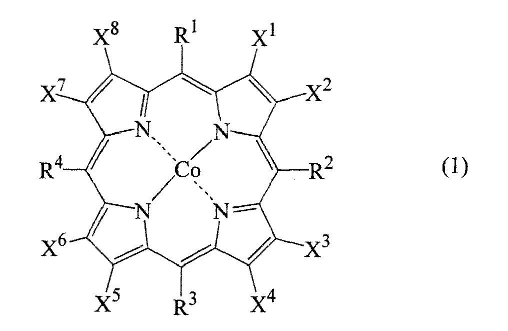 wherein X¹ to X⁸ independently represent a hydrogen atom, a halogen atom, a nitro group, a cyano group, a hydrocarbon group, a halogenated hydrocarbon group or a sulfonyl group, provided that at least one of X¹ to X⁸ is a halogen atom, and R¹ to R⁴ independently represent a hydrogen atom, a nitro group, a cyano group, a hydrocarbon group or a halogenated hydrocarbon group.

2. The process according to claim 1, wherein X¹ to X⁸ in the formula (1) are halogen atoms.

3. The process according to claim 1 or 2, wherein the cycloalkane is cyclohexane.
